# EUROPEAN PATENT SPECIFICATION

(11) **EP 3 144 674 B1**
(45) Date of publication and mention of the grant of the patent: **02.12.2020**
(21) Application number: 15185933.7
(22) Date of filing: 18.09.2015
(51) Int. Cl.: G01N 33/483

(54) **UPSIDE-DOWN MICROELECTRODE ARRAY FOR SINGLE- AND MULTICELL CULTURES**
UPSIDE-DOWN-MIKROELEKTRODENANORDNUNG FÜR EINZEL- UND MEHRZELLENKULTUREN
RÉSEAU DE MICROÉLECTRODES INVERSÉ POUR CULTURES UNI- ET MULTICELLULAIRES

(43) Date of publication of application: 22.03.2017
(73) Proprietor: Hochschule Kaiserslautern University of Applied Sciences, 67659 Kaiserslautern (DE)
(72) Inventor: SCHÄFER, Karl-Herbert, 66495 Kirkel Altstadt (DE); RABE, Holger, 35510 Butzbach (DE); SCHLÜTER, Sebastian, 66482 Zweibrücken (DE)
(74) Representative: Patentanwälte Dr. Keller, Schwertfeger Partnerschaft mbB

(56) References cited:
- US-A- 6 051 422
- US-A1- 2014 274 796
- US-B1- 6 377 057
- DUPORT S ET AL: "A metallic multisite recording system designed for continuous long-term monitoring of electrophysiological activity in slice cultures", BIOSENSORS AND BIOELECTRONICS, ELSEVIER BV, NL, vol. 14, no. 4, 30 April 1999 (1999-04-30) , pages 369-376, XP002211652, ISSN: 0956-5663, DOI: 10.1016/S0956-5663(99)00015-9
- MORIN F ET AL: "Constraining the connectivity of neuronal networks cultured on microelectrode arrays with microfluidic techniques: A step towards neuron-based functional chips", BIOSENSORS AND BIOELECTRONICS, ELSEVIER BV, NL, vol. 21, no. 7, 15 January 2006 (2006-01-15), pages 1093-1100, XP024961388, ISSN: 0956-5663, DOI: 10.1016/J.BIOS.2005.04.020 [retrieved on 2006-01-15]
- C?dric Bathany ET AL: "A microfluidic platform for measuring electrical activity across cells", Biomicrofluidics, 1 September 2012 (2012-09-01), page 34121, XP055325610, United States DOI: 10.1063/1.4754599 Retrieved from the Internet: URL:http://www.mae.buffalo.edu/printable/m icrofluidic_platform.pdf

## Description

The present invention relates to microelectrode arrays (MEAs) comprising an electrode carrier which supports a plurality of microelectrodes disposed on the upper surface of the electrode carrier and a cover slip with electroconductive cells adhered thereon. The microelectrodes are configured to transmit or receive an electrical signal to or from the electroconductive cells. The MEA also comprises at least one chamber which accommodates the electrode carrier and the cover slip carrying the electroconductive cells. The invention further relates to a method for extracellular simulation or recording of electrical activity of electrogenic cells in which the cover slip carrying the electroconductive cells is placed upside-down onto the electrode carrier such that the electroconductive cells are in electrical contact with the plurality of microelectrodes.

Microelectrode arrays (MEAs) are regularly used for an analysis of electrically active cells, such as cardiomyocytes, neurons or heart cells. Substrate-integrated microelectrode arrays are matured systems for extracellular recordings and stimulation of electrically active (eletroconductive) cells. The signals received by an MEA provide a deeper understanding of the relationships between the functional connectivity-map of neuronal circuits and their physiological or pathological functions. In vitro, MEAs usually contain a plurality of electrodes in order to gain the required information from the cells. Extracellular recording of single-cell activity is usually achieved with planar electrodes which are in contact with individual cells. In such systems, the cell body partially covers the electrode surface, and a free electrode area is in contact with a culturing solution (ionic solution). Thus, the cell membrane is partially in contact with the electrode, whereas the remaining part of the membrane faces the culturing solution or the culture substrate. The electrode is coupled to an amplifier which receives and amplifies the signals obtained from the cells. The amplifier is connected to the contacting lane and records the sum of the potentials at the surface of the free electrode and the surface of the electrode covered by the membrane.

The MEA approach cannot only be used for single cells, but also for multiple cells or tissues. The electrical activity spreads within the cellular compartments and from cells to cells via synaptic connections. This spread of excitation within cells and tissue is associated with a flow of ionic current through the extracellular fluid. The recordings usually exhibit slow field potentials and fast spikes arising from action potentials.

Microelectrodes can also be used for extracellular electrical stimulation by applying current or voltage impulses to the electroconductive cells. Microelectrodes utilized for extracellular electrical stimulation should offer a high-charge injection capacity. The standard MEA electrode is usually made of TiN using plasma-enhanced chemical vapour deposition (PECVD). The insulator is usually made of silicon nitride (Si₃N₄).

From the first development of microelectrode arrays in the early 1970s, the layout of arrays has been changed and adapted for a particular application. Examples of such MEA layouts are Standard Line MEA, Thin MEA, Flex MEA, High-Density MEA or Perforated MEA for tissue recording. Depending on the layout, the number and deposition of the electrodes on the carrier is significantly different. Furthermore, efforts were made for up-scaling MEA systems in order to be applicable for pharmaceutical applications, drug testing and research on neuronal or cardiac cells.

The use of MEAs in high-throughput applications is currently demanding and cost-effective because MEA chips are timely blocked during the cell growth phase. This is because electroconductive cells are directly cultured on a substrate-integrated planar extracellular electrode. The shape of electrodes can differ to provide an optimum electrical contact with the electroconductive cells. Nanopillar electrodes can extend into a cultured electroconductive cell, but do not penetrate the plasma membrane. After the application of an electroporating stimulus, the nanopillar gains access to the cytoplasm. An array of nanopillars that penetrate the plasma membrane are in direct physical contact with the cytosol. However, stimulation of cells by substrate-integrated electrodes (either passive metal electrodes or FETs) often involves undesired electrochemical reaction products at the electrode interface and consequently damage to the cells that go beyond transient electroporation. In addition, cells cultured on MEAs suffer from exhausting culture medium, and toxic products that may interfere with the stimulation or measurement of electrical signals. Furthermore, individual MEAs are blocked for the culture time and cannot be used for other purposes, because the elecctroconductive cells require sufficient time to adhere to the electrode surface and to establish cell-junctions.

Therefore, it takes a long preparation time before an MEA can actually be used for stimulation or recording purposes. Consequently, the number and quality of MEAs must be carefully calculated and determined in order to obtain high qualitative MEAs for the subsequent application.

MEAs become unusable because of the ongoing consumption of the cell culture medium and possibly dying cells. Furthermore, MEAs have to be used in parallel in order to obtain a significant number of data within the test period. A further problem arises in particular in cultures of primary neurons which very often are not optimally placed or grown on the MEA, thus requiring a high number of MEAs to be prepared. In addition, the replacement of none-functional MEAs with working MEAs results in high costs for individual function analyses and for manufacture of the MEAs. Furthermore, it becomes more difficult to compare results because the culturing conditions of individual cell types must be adapted to the currently applied MEA method.

Systems and methods have been described for electrical contacting biological cells such the ones in DE 10 2011 120 394 A1.

EP 2 278 313 A1 described electrodes, methods and apparatuses comprising microelectrode arrays. The array of microelectrodes is disposed on a flexible substrate such as a flexible polymeric material.

CN 103031246 A describes a microelectrode array chip for multi-parameter detection of nerve cells and a preparation method thereof, relating to a sensor technology. The chip consists of seven parts, including an insulating base, a microelectrode array, counter electrodes, reference electrodes, electrode leads and insulating material. The nerve cells are cultivated on the surface of modified working electrodes. The microelectrode array chip is used for detecting electrophysiological signals of the nerve cells and of neurotransmitters such as dopamine or acetyl choline.

TW2009 16752 A describes a cellular microarray, which has a substrate, multiple first conductive lines, multiple second conductive lines and multiple feather-like electrode units arranged on the surface of the substrate in an array. The micro array chip can adhere the cells rapidly and uniformly and thereby increasing the throughout in the manufacturing process and reducing costs.

A related microelectrode array and a related method for extracellular stimulation or recording of electrical activity of electrogenic cells is disclosed in DUPORT S ET AL: "A metallic multisite recording system designed for continuous long-term monitoring of electrophysiological activity in slice cultures",BIOSENSORS AND BIOELECTRONICS, ELSEVIER BV, NL, vol. 14, no. 4, 30 April 1999 (1999-04-30), pages 369-376

It is object of the present invention to provide an improved multielectrode array and a method which minimizes the blocking time of MEAs, and that increases the number of cultures to be measured using single or multi cell MEAs.

The problem underlying the present invention is solved by a microelectrode array as claimed in claim 1. Preferred embodiments are subject of the dependent claims.

The basic idea of the invention is to let electroconductive cells grow onto conventional cover slips (such as glass cover slips or cover slips made of synthetic material) under controlled cell culture conditions. Using this approach, the cells can be cultured under similar conditions and do not block any MEA. The invention further suggests placing the cover slips carrying the electroconductive cells upside-down onto the microelectrodes of the MEA. The placement of the cover slip onto the electrodes usually does not require much pressure, and a damage of cells by strong compression should be avoided. Therefore, the invention suggests that the plurality of microelectrodes is disposed on the upper surface of an electrode carrier which is accommodated in a culture chamber. The chamber accommodates both the electrode carrier and the cover slip carrying the electroconductive cells. The placement of the cover slip upside-down onto the electrodes allows for an efficient electrical contact between the cell membranes of the electroconductive cells and the surface of the microelectrodes. The invention further suggests that the bottom of the cover slip and the upper surface of the electro carrier form a flow channel within the chamber for transporting fluids to or from the electroconductive cells. The flow channel thus generates a culture space for the electroconductive cells within the chamber.

The microelectrode array of the invention comprises a micro-channel structure and conduits that allow a circulation of cell culture medium embedding the cover slip carrying the elctroconductive cells.

The microelectrode array of the invention thus allows increasing the number of parallel measurements such as stimulations or recordings of electrical signals to or from the electrically active cells. In the microelectrode arrays of the prior art, the cells had to grow on the electrodes to measure the activities of cells. In order to obtain an optimal adherence of the cells on the surface of the electrode, a coating with biological molecules such as polylysine or laminin was mandatory. This coating, however, also changed the impedance of the system. The microelectrode array of the invention avoids these disadvantages and allows a standardization of the reaction conditions, thus improving the quality of the data. The MEA of the invention can be used to evaluate the influence of biologically active compounds or toxins on neuronal cells or cardiac cells, membrane oscillations of β-cells or the detection of signal molecules released from the cells. Using the cover slips of the present invention, the cells can directly grow thereon, and it is no longer necessary to occupy the surface of the electrodes with cells. The cover slips of the invention also allow dissociated cells to establish their cell-cell junctions within the cell tissue in order to regain their function. By the standardization of the population conditions of the electroconductive cells on the cover slips of the invention, experiments can be better compared.

The chamber of the microelectrode array incorporates the electrode carrier and the cover slip. The bottom side of the cover slip and the upper surface of the electrode carrier form the flow channel for the culture media. The flow channel, as part of a perfusion system, allows a continuous supply with cell culture media or test solutions. Furthermore, the volume of the culture media can be significantly reduced as the amount surrounding the cells is minimized.

The present invention demonstrates that electrical signals can be transmitted or received from the electroconductive cells which are attached with the microelectrodes of the microelectrode array in the upside-down approach as described above. The pressure used to attach the cells onto the electrodes can differ and must be adjusted accordingly. In a preferred embodiment, the bottom side of the cover slip, which bears the electroconductive cells, further comprises one or more spacer elements to maintain a distance (A) between the bottom side of the cover slip and the upper surface of the electrode carrier to form the flow channel. The length of the space elements can vary and will depend on the cell type and thickness of the cell layer grown on the cover slip. Neuronal or cardiac cells of different dimensions require different distances, and therefore the spacers must be adjusted accordingly. Greater distances are usually necessary using neuronal cells (5 µm) as compared to cardiac muscle cells, which are rather planar (1-2 µm). The spacer elements provide a distance between the bottom side of the cover slip and the upper surface of the electrode carrier range in the range from > 0 - 10 µm, preferably between 0,5 and 6 µm. Spacer elements with a length of 0.5, 1, 2, 4, 5 and 6 µm are preferred n the present invention.

The populated cell-carrying cover slips are attached to the microelectrodes with a defined pressure, and signals can be received or transmitted by testing different compression pressures. To apply some pressure, the invention further comprises a plug that can be inserted in the interior of the chamber which also helps to avoid a displacement of the cover slip relative to the microelectrodes.

The electrode carrier carries a plurality of microelectrodes which are suitable to transmit or receive an electrical signal to or from the electroconductive cells grown on the cover slip. The microelectrodes can be placed, for example, on a glass carrier. In a preferred embodiment, the bottom of the chamber builds the electrode carrier.

In order to supply nutrients and/or test substances to the cells, or in order to analyse the cell culture medium upon stimulation of the electroconductive cells with an electrical signal, a perfusion system is established, which is also part of the present invention. According to this variant, the flow channel of the chamber is fluidly connected to a supply conduit and a discharge conduit. The supply conduit can transport the cell culture medium into the flow channel in order to supply the cells with cell culture medium. The discharge conduit allows the removal of the culture medium from the flow channel. A continuous flow can be established where cell culture medium circulates between the supply conduit and the discharge conduit over the flow channel. The supply with new culture medium or test solution can also be triggered in intervals, which will depend on the layout of the experiment to be conducted. The perfusion of fresh cell culture medium into the chamber allows the cells to maintain their function in order to receive or transmit electrical signals to the microelectrodes. After the measurements, the cells can be immunhistochemically stained and further analysed, for example by using a life-dead assay, or the cells can be investigated using electro-optical methods.

The use of the above-mentioned microfluidic channels that consist of a supply conduit, a flow channel and a discharge conduit allows a constant perfusion of the cells with media over a defined period. The flow is preferably adjusted in order to achieve a constant flow at minimum flow rates. In a preferred embodiment, the exchange of cell culture medium can be increased by using perforated cover slips that comprise one or more perforations forming a duct through the cover slip in order to transport the fluid. Preferably, the perforations are formed at the outer area of the cover slip to transport the fluids into the flow channel formed by the cover slip and the electrode carrier. The fluid flow is conducted via the perforations of the cover slips into the flow channel, for example by using differential pressures. This can be achieved, for example, by applying low pressure or vacuum pressure within the flow channel to suck the fluid through the perforations, followed by a withdrawal of the fluid into the discharge conduit. The flow is adjusted in order to secure an optimum survival of the electroconductive cells. The use of microfluidic channels significantly reduces the amount of cell culture medium used to conduct the measurements. The system also allows auto-connective activity of the cells, which permanently release cytokines, hormones or transmitters. This situation of the microelectrode array of the invention is comparable with the in-vivo conditions found within tissue, resulting in an optimum cell growth in the microelectrode array of the invention. Furthermore, the small volumes of fluid allow high concentrations of these substances, which can be better detected, e.g. by multiplex ELISA measurements.

In order to improve the electrical conductivity and contact between the electroconductive cells and microelectrodes, the electrodes can be made of or coated with a conductive polymer such as 3,4-ethylenedioxythiophene. This polymer has a suitable electrical and ionic conductivity when used in the microelectrode array of the invention. It provides a surface which allows an improved adherence of the cells. The microelectrodes used in the present invention have a diameter between 5 and 50 µm, preferably between 10 and 30 µm. The polymer can also be used for the coating of conventional electrodes in order to improve their conductivity and surface structure.

Furthermore, electrically active cells can be stimulated with neuronal transmitters in the vicinity of the electrodes to improve adherence of the cells. Preferably, Al₂O₃ in connection with oxalic acid is used for this purpose, which introduces nanopores at the electrodes. A thin layer of Al₂O₃ (e.g. 40 - 80 µm) is applied onto the electrodes using a micro-spotter. In order to produce suitable nanopores, different voltages and/or concentrations of oxalic acid are used. The cells are electrooptically analysed. The method is used in order to reduce homogenous nanopores with a diameter between 15 and 25 µm. The nanopores are filled with new transmitters to attract neurons. Since neuron transporters usually have an ionic conductivity, this feature can be used to locate the neurotransmitters into the nanopores by applying a complementary electrical field. Preferably, the neuron transmitters are dissolved in a physiological ionic solution in order to increase viscosity. This allows an improved electrical contact and a prolonged persistence of the neurotransmitter within the nanopores, even if surrounded by liquid media.

In a variant, three-dimensional (3-D) electrodes can be used in addition or alternatively to planar electrodes. The use of three-dimensional electrodes allows for an intracellular transmission or reception of signals to or from the cells because of the increased surface of the electrodes. Pharmacological investigations on cell lines are usually only of limited value. Preferably, the culture should resemble in-vivo conditions in order to be suitable for biological or pharmaceutical applications. Electroconductive cells such as nerve cells of the peripheric nerve system or cardiac muscle cells interact with each other and are also influenced by a number of factors, such as intestinal motility or resorption forces of the gastro-intestinal microbiome (intestinal flora).

In order to simulate the conditions where indirect factors influence the electroconductive cells, a multi-chamber system has been established as part of the present invention. For this purpose, the perfusion system is extended by serially connecting a number of chambers and by establishing a flow of fluid between the chambers. The fluid that is transported through the chambers will be conditioned in dependency of the cell lines, cell interactions and cell culture conditions. Furthermore, substances can be tested which are present in the fluid in order to evaluate their effect on different cell types in a network. For this purpose, the electrical signals are preferably independently measured from each chamber and evaluated.

For example, spinal ganglion cells can be cultured in one chamber and cardiac muscle cells can be cultured in an adjacent chamber. The multi chamber arrays of the invention allow to determine how an intoxication of the spinal ganglion cells affects the activity of the cardiac muscle cells. In other chambers, intestinal nerve cells can be cultivated and the effect of intestine bacteria can be investigated within the MEA chamber. The bacteria can be encapsulated in capsules made of alginate and then be populated by the perfusion system of the invention.

The microelectrode array can also be used for cyclic voltametric measurements of the release and concentration of different neurotransmitters (e.g. dopamine). The multi-chamber approach can also be used in a multi- well-plate which contains a plurality of wells, wherein each well defines a chamber equipped with an electrode carrier which supports a plurality of microelectrodes and a cover slip to which the electroconductive cells are adhered. The microelectrodes extend into the flow channel that is formed between the cover slip and the electrode carrier. Most importantly, the cover slips are placed upside-down onto the electrodes, i.e. the electroconductive cells are faced towards the electrodes. By applying a certain degree of pressure, the cells contact the surface of the electrodes in order to transmit or receive electrical signals. The chambers of the multi-chamber system are preferably connected with the supply conduit and/or the discharge conduit.

In order to allow for an exchange of fluid between the chambers, a supply adapter is used, consisting of an insert to be inserted into the chamber and a top part which integrates a tube to transport the fluid into or from the chamber. Preferably, each chamber comprises a separate supply adapter. The insert preferably has the same or a slightly smaller diameter than the diameter of the chamber and also allows the application of a certain pressure onto the back surface of the cover slip in order to press the cover slip with the underneath adhering electroconductive cells towards the electrodes to attach the cells to the surface of the electrodes.

Several chambers are connected to form a multi-chamber system, and each chamber is equipped with an individual supply adapter. At least one chamber further comprises a joint plug with a therein integrated fluid chamber which is fluidly connected with the tube of the supply adapter of an adjacent chamber via a connecting tube. This construction allows the exchange of fluid from one chamber to the adjacent chamber. The continuous or irregular fluid flow is achieved within a chamber or between the chambers of a multi-chamber system using differential pressure. The multi-chamber system can further comprise a pump which circulates the fluid from one chamber to an adjacent chamber.

In a variant, the supply adapter is held by a spacer ring to maintain a distance (A) between the bottom side of the cover slip and the upper side of the electrode carrier to form the flow channel. Depending on the thickness of the spacer ring, the distance can be adjusted accordingly. In a preferred embodiment, more than one spacer ring are mounted onto a cover plate, each spacer ring is ready for assembly of a supply adapter and, if applicable, a joint plug. Furthermore, the supply adapter can be held by a gripper, which comprises an attachment part which attaches the insert of the supply adapter. In a variant, a spacer ring frames the supply adapter and the gripper attaches the spacer ring instead of the supply adapter.

This modular construction of the multi-chamber system of the invention allows the cultivation of different cell types or a number of cells of the same cell type in different chambers. The chambers are preferably connected to one perfusion system that serially or individually supplies the chambers and the therein formed culture spaces with cell culture medium. Therefore, the influences of certain culture conditions or substances can be evaluated for each cell type present in the individual chambers.

The present invention also relates to a method for extracellular stimulation of recording electrical activity of electrogenic cells, comprising the following steps:
a. providing a plurality of microelectrodes on the upper service of an electrode carrier,
b. providing electroconductive cells or cell tissue adhered on a cover slip,
c. contacting the electroconductive cells by the microelectrodes, wherein the microelectrodes transmit or receive electrical signals to or from the electroconductive cells,
d. placing the cover slip carrying the electroconductive cells upside-down onto the electrode carrier such that the electroconductive cells are in electrical contact with the plurality of microelectrodes,
e. forming a flow channel within a chamber for transporting fluids to or from the electroconductive cells by keeping a distance (A) between the bottom side of the cover slip and the upper surface of the electrode carrier.

Preferably, the electrical activity of the electroconductive cells is measured by a microelectrode array as described above.

The following examples and embodiments will explain the invention in more detail.

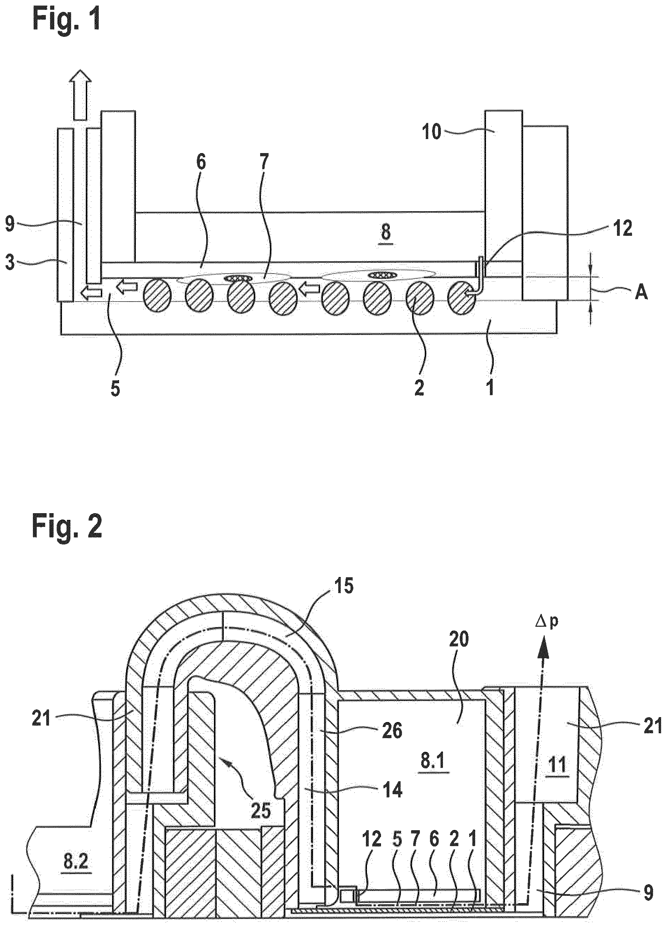

Fig. 1 shows a microelectrode array of the present invention, comprising an electrode carrier 1, which supports a plurality of microelectrodes 2 disposed on the upper surface of the electrode carrier 1. The electroconductive cells 7 are adhered to the surface of a cover slip 6. The cover slip 6 carrying the electroconductive cells 7 is placed upside-down onto the electrode carrier 1 such that the electroconductive cells 7 are in electrical contact with the plurality of microelectrodes 2. A chamber 8 accommodates the electrode carrier 1 and the cover slip 6 carrying the electroconductive cells 7. The bottom side of the cover slip 6 and the upper surface of the electrode carrier 1 form the flow channel 5 within the chamber 8 for transporting fluids to or from the electroconductive cells 7.

In the embodiment shown in Fig. 1, the cover slip 6 also comprises perforations 12 formed at the outer area of the cover slip 6 for transporting the fluids into the flow channel 5 formed by the cover slip 6 and the electrode carrier 1. The flow channel 5 is connected to a supply conduit 14 and a discharge conduit 9. This allows a continuous flow at very low flow rates of the provided cell culture medium. The circulation of fluid can be continuous or conducted in intervals. In the embodiment shown, the chamber 8 comprises chamber walls 10 and a bottom plate which holds the electrode carrier 1.

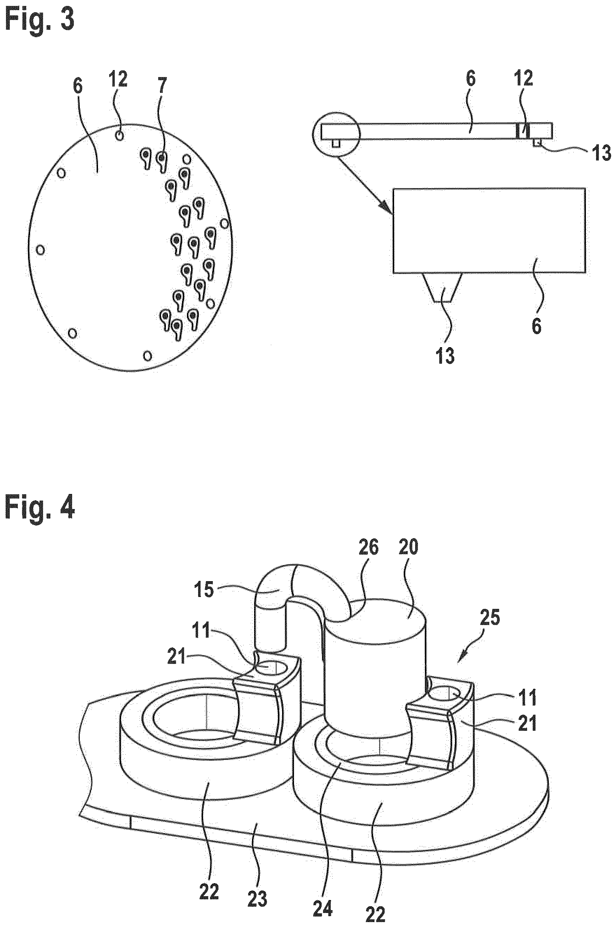

Fig. 2 shows the fluidic flow through the chambers of a multi-chamber system. Fluid is guided from one chamber 8.1 into an adjacent chamber 8.2. Each chamber 8 is equipped with an individual supply adapter 25, wherein the chamber 8.1 further comprises a joint plug 20 with a therein integrated fluid channel 26 (see Fig. 3). The supply adapter 25 consists of an insert 24 and a top part 21, which integrates a tube 11 to transport the fluid into or from the chamber 8. The flow channel 5 is fluidly connected to a supply conduit 14 and a discharge conduit 9. The chambers 8 are fluidly connected using a connecting tube 15, wherein one end is connected with the fluid channel 26 which is integrated into the joint plug 20, and the other end is connected with the tube 11 of the supply adapter 25 of the adjacent chamber 8.2.

Fig. 3 shows a cover slip 6 which contains a plurality of perforations 7 for transporting the fluids into the flow chamber 5 formed by the cover slip 6 and the electrode carrier 1. The cover slip also comprises space elements 13 to maintain a distance (A) between the bottom side of the cover slip and the upper surface of the electrode carrier 6 to form the flow channel 5 (see Fig. 2). The electroconductive cells 7 grow on the surface of the cover slip 6. The perforated cover slip 6 is placed upside-down onto the electrodes within the chamber 8.

Fig. 4 shows a multi-chamber connection comprising two supply adapters 25, each consisting of an insert 24 to be inserted into a chamber 8, and a top part 21 which integrates the tube 11. The joint plug 20 integrates a fluid channel 26 which is fluidly connected with the tube 11 of a supply adapter 25 via connecting tube 15. The supply adapter 25 is surrounded by a spacer ring 22. The top part of the supply adapter 25 sits on the other part of the spacer ring 22 and provides a certain distance (A) when mounted into a chamber 8. If a small distance (A) is required between the bottom side of the cover slip 6 and the upper surface of the electrode carrier 1 (e.g. higher pressure required), a smaller spacer ring 22 can be used. By using different types of the spacer ring 22, the distance and therefore the height of the flow channel 5 within the chamber 8 can be adjusted. In the shown embodiment, two supply adapters 25, each surrounded by a spacer ring 22, are mounted onto a cover plate 23.

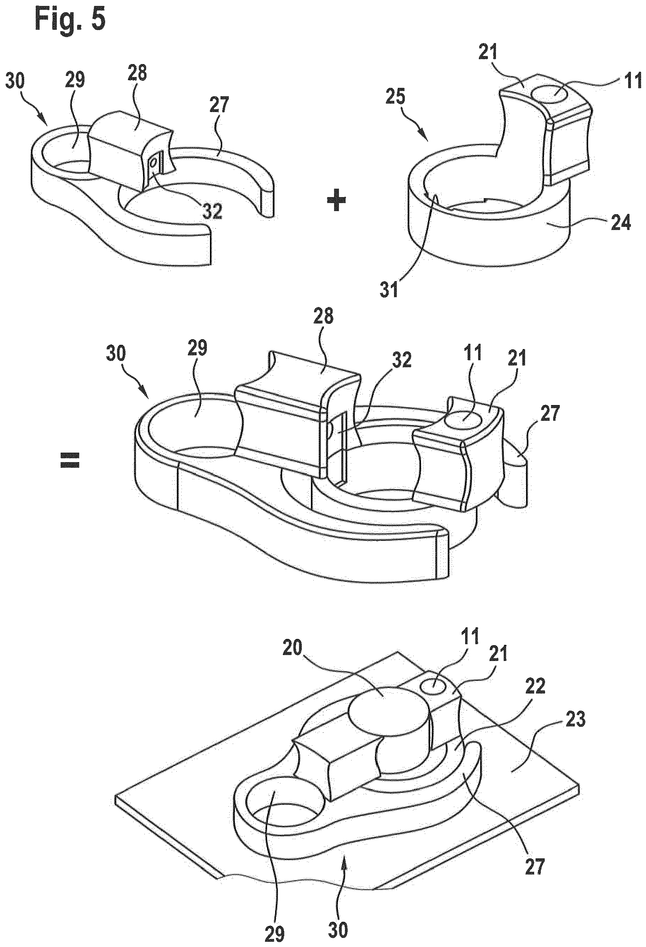

Fig. 5 shows a further embodiment which extends the MEA of the invention to a multi-chamber system. The supply adapter 25 is held by a gripper 30 which comprises an attachment part 27 which attaches the insert 24 of the supply adapter 25. Furthermore, a spacer ring frames the supply adapter 25, such that the gripper 30 attaches the spacer ring 22. Again, the gripper 30, the supply adapter 25 and the spacer ring 22 can be mounted on a cover plate 23.

Fig. 6 shows the results of the experiment using neuronal cells (to be completed by the inventors).

The MEA of the invention can be used to measure the electrophysiological signals of electroconductive cells, such as field potential, field potential profiles, field potential duration, QT intervals, conduction velocity, electrophysical profile, spontaneous beating rate, re-entrant wave patterns, and impendence.

## Claims

1. A microelectrode array, comprising
a. an electrode carrier (1) which supports a plurality of microelectrodes (2) disposed on the upper surface of the electrode carrier (1),
b. a cover slip (6) with a surface to which electroconductive cells (7) are adhered, wherein the microelectrodes (2) are configured to transmit or receive an electrical signal to or from the electroconductive cells (7),
c. a chamber (8), which accommodates the electrode carrier (1) and the cover slip (6) carrying the electroconductive cells (7),
**characterized in that** the cover slip (6) carrying the electroconductive cells (7) is placed upside-down onto the electrode carrier (1) such that the electroconductive cells (7) are faced towards the microelectrodes (2) and that the electroconductive cells (7) are in electrical contact with the plurality of microelectrodes (2) to receive and transmit electrical signals, wherein the bottom side of the cover slip (6) which bears the electroconductive cells (7) and the upper surface of the electrode carrier (1) form a flow channel (5) within the chamber (8) for transporting fluids to or from the electroconductive cells (7).

2. The microelectrode array according to claim 1, wherein the bottom of the chamber (8) builds the electrode carrier (1).

3. The microelectrode array according to claim 1, wherein the flow channel (5) of the chamber (8) is fluidly connected to a supply conduit (14) and a discharge conduit (9).

4. The microelectrode array according to claim 3, wherein the supply conduit (14) and/or the discharge conduit (9) connects adjacent chambers (8) to form a multi-chamber system, wherein each chamber (8) is individually equipped with an electrode carrier (1), a cover slip (6) and a flow channel (5) formed by the bottom side of the cover slip (6) and the upper surface of the electrode carrier (6).

5. The microelectrode array according to any one of claims 1 to 4, wherein the bottom side of the cover slip (6) further comprises spacer elements (13) to maintain a distance (A) between the bottom side of the cover slip (6) and the upper surface of the electrode carrier (6) to form the flow channel (5).

6. The microelectrode array according to any one of claims 1 to 5, wherein the cover slip (6) comprises one or more ducts (12) formed at the outer area of the cover slip (6) for transporting the fluids into the flow channel (5) formed by the cover slip (6) and the electrode carrier (1).

7. The microelectrode array according to any one of claims 1 to 6, wherein the chamber (8) comprises a supply adapter (25) consisting of an insert (24) to be inserted into the chamber (8) and a top part (21), which integrates a tube (11) to transport the fluid into or from the chamber (8).

8. The microelectrode array according to claim 7, wherein several chambers (8) are connected to form a multi-chamber system, and each chamber (8) is equipped with an individual supply adapter (25), wherein at least one chamber (8.1) further comprises a joint plug (20) with a therein integrated fluid channel (26) which is fluidly connected with the tube (11) of the supply adapter (25) of an adjacent chamber (8.2) via a connecting tube (15).

9. The microelectrode array according to claim 8, wherein the multi-chamber system further comprises a pump which circulates the fluid from one chamber (8.1) to an adjacent chamber (8.2).

10. The microelectrode array according to anyone of claims 7 to 9, wherein the supply adapter (25) is held by a spacer ring (22) to maintain a distance (A) between the bottom side of the cover slip (6) and the upper surface of the electrode carrier (6) to form the flow channel (5).

11. The microelectrode array according to claim 10, wherein more than one spacer ring (22) are mounted onto a cover plate (23), each spacer ring (22) is ready for assembly of a supply adapter (25) and, if applicable, a joint plug (20).

12. The microelectrode array according to claim 7, wherein the supply adapter (25) is held by a gripper (30) which comprises an attachment part (27) which attaches the insert (24) of the supply adapter (25).

13. The microelectrode array according to claim 12, wherein a spacer ring (22) frames the supply adapter (25), and the gripper (3) attaches the spacer ring (22).

14. A method for extracellular stimulation or recording of electrical activity of electrogenic cells, comprising the following steps:
a. providing a plurality of microelectrodes on the upper service of an electrode carrier,
b. providing electroconductive cells or cell tissue adhered on a cover slip,
c. contacting the electroconductive cells by the microelectrodes, wherein the microelectrodes transmit or receive electrical signals to or from the electroconductive cells,
d. placing the cover slip carrying the electroconductive cells upside-down onto the electrode carrier such that the electroconductive cells are faced towards the microelectrodes and that the electroconductive cells are in electrical contact with the plurality of microelectrodes to transmit or receive electrical signals,
e. forming a flow channel within a chamber for transporting fluids to or from the electroconductive cells by keeping a distance (A) between the bottom side of the cover slip which carries the electroconductive cells and the upper surface of the electrode carrier.

15. The method according to claim 14, wherein the electrical activity of the electroconductive cells is measured by a microelectrode array according to any one of claims 1 to 13.

## Patentansprüche

1. Mikroelektroden-Array, umfassend
a. einen Elektrodenträger (1), der eine Vielzahl von Mikroelektroden (2) trägt, die auf der oberen Oberfläche des Elektrodenträgers (1) angeordnet sind,
b. ein Deckglas (6) mit einer Oberfläche, an der elektrisch leitende Zellen (7) haften, wobei die Mikroelektroden (2) so konfiguriert sind, dass sie ein elektrisches Signal zu oder von den elektrisch leitenden Zellen (7) übertragen oder empfangen,
c. eine Kammer (8), in der der Elektrodenträger (1) und das Deckglas (6) mit den elektrisch leitfähigen Zellen (7) untergebracht sind,
**dadurch gekennzeichnet, dass** das die elektrisch leitenden Zellen (7) tragende Deckglas (6) mit der Oberseite nach unten auf den Elektrodenträger (1) gelegt ist, so dass die elektrisch leitenden Zellen (7) den Mikroelektroden (2) zugewandt sind, und dass die elektrisch leitenden Zellen (7) in elektrischem Kontakt mit der Vielzahl von Mikroelektroden (2) stehen, um elektrische Signale zu empfangen und zu übertragen, wobei die Unterseite des Deckglases (6), das die elektrisch leitfähigen Zellen (7) trägt, und die Oberseite des Elektrodenträgers (1) einen Strömungskanal (5) innerhalb der Kammer (8) zum Transport von Fluiden zu oder von den elektrisch leitfähigen Zellen (7) bilden.

2. Mikroelektroden-Array nach Anspruch 1, wobei der Boden der Kammer (8) den Elektrodenträger (1) bildet.

3. Mikroelektrodenanordnung nach Anspruch 1, wobei der Strömungskanal (5) der Kammer (8) mit einer Zufuhrleitung (14) und einer Abfuhrleitung (9) in Fluidverbindung steht.

4. Mikroelektrodenanordnung nach Anspruch 3, bei der die Zufuhrleitung (14) und/oder die Abfuhrleitung (9) benachbarte Kammern (8) zu einem Mehrkammersystem verbindet, wobei jede Kammer (8) einzeln mit einem Elektrodenträger (1), einem Deckglas (6) und einem durch die Unterseite des Deckglases (6) und die Oberseite des Elektrodenträgers (1) gebildeten Strömungskanal (5) ausgestattet ist.

5. Mikroelektrodenanordnung nach einem der Ansprüche 1 bis 4, wobei die Unterseite des Deckglases (6) ferner Abstandselemente (13) zur Aufrechterhaltung eines Abstands (A) zwischen der Unterseite des Deckglases (6) und der Oberseite des Elektrodenträgers (1) zur Bildung des Strömungskanals (5) aufweist.

6. Mikroelektrodenanordnung nach einem der Ansprüche 1 bis 5, wobei das Deckglas (6) einen oder mehrere Kanäle (12) aufweist, die im Außenbereich des Deckglases (6) ausgebildet sind, um die Fluide in den durch das Deckglas (6) und den Elektrodenträger (1) gebildeten Strömungskanal (5) zu transportieren.

7. Mikroelektrodenanordnung nach einem der Ansprüche 1 bis 6, wobei die Kammer (8) einen Versorgungsadapter (25) aufweist, der aus einem in die Kammer (8) einzusetzenden Einsatz (24) und einem Oberteil (21) besteht, in dem ein Kanal (11) integriert ist, um das Fluid in die Kammer (8) hinein oder aus ihr heraus zu transportieren.

8. Mikroelektroden-Array nach Anspruch 7, bei dem mehrere Kammern (8) zu einem Mehrkammersystem verbunden sind und jede Kammer (8) mit einem individuellen Versorgungsadapter (25) ausgestattet ist, wobei mindestens eine Kammer (8.1) ferner einen Verbindungsstopfen (20) mit einem darin integrierten Fluidkanal (26) aufweist, der über einen Verbindungskanal (15) mit dem Kanal (11) des Versorgungsadapters (25) einer benachbarten Kammer (8.2) in Fluidverbindung steht.

9. Mikroelektrodenanordnung nach Anspruch 8, wobei das Mehrkammersystem ferner eine Pumpe umfasst, die das Fluid von einer Kammer (8.1) zu einer benachbarten Kammer (8.2) zirkuliert.

10. Mikroelektrodenanordnung nach einem der Ansprüche 7 bis 9, wobei der Versorgungsadapter (25) durch einen Abstandsring (22) gehalten wird, um einen Abstand (A) zwischen der Unterseite des Deckglases (6) und der Oberseite des Elektrodenträgers (1) zur Bildung des Strömungskanals (5) aufrechtzuerhalten.

11. Mikroelektroden-Array nach Anspruch 10, bei dem mehr als ein Abstandsring (22) auf einer Deckplatte (23) montiert ist, wobei jeder Abstandsring (22) zur Montage eines Versorgungsadapters (25) und gegebenenfalls eines Verbindungssteckers (20) bereit ist.

12. Mikroelektrodenanordnung nach Anspruch 7, wobei der Versorgungsadapter (25) von einem Greifer (30) gehalten wird, der ein Befestigungsteil (27) aufweist, das den Einsatz (24) des Versorgungsadapters (25) verbindet.

13. Mikroelektrodenanordnung nach Anspruch 12, wobei ein Abstandsring (22) den Versorgungsadapter (25) umrahmt und der Greifer (30) den Abstandsring (22) befestigt.

14. Verfahren zur extrazellulären Stimulation oder Aufzeichnung der elektrischen Aktivität von elektrogenen Zellen, umfassend die folgenden Schritte:
a. Bereitstellung einer Vielzahl von Mikroelektroden auf der Oberfläche eines Elektrodenträgers,
b. Bereitstellung von elektrisch leitfähigen Zellen oder Zellgewebe, das auf einem Deckglas haftet,
c. Kontaktieren der elektrisch leitfähigen Zellen durch die Mikroelektroden, wobei die Mikroelektroden elektrische Signale zu oder von den elektrisch leitfähigen Zellen senden oder empfangen,
d. Auflegen des Deckglases, das die elektrisch leitenden Zellen trägt, mit der Oberseite nach unten auf den Elektrodenträger, so dass die elektrisch leitenden Zellen den Mikroelektroden zugewandt sind und dass die elektrisch leitenden Zellen in elektrischem Kontakt mit der Vielzahl von Mikroelektroden stehen, um elektrische Signale zu übertragen oder zu empfangen,
e) Bilden eines Strömungskanals innerhalb einer Kammer zum Transport von Flüssigkeiten zu oder von den elektrisch leitfähigen Zellen, indem ein Abstand (A) zwischen der Unterseite des Deckglases, der die elektrisch leitfähigen Zellen trägt, und der Oberseite des Elektrodenträgers eingehalten wird.

15. Verfahren nach Anspruch 14, bei dem die elektrische Aktivität der elektrisch leitfähigen Zellen mit einer Mikroelektrodenanordnung nach einem der Ansprüche 1 bis 13 gemessen wird.

## Revendications

1. Réseau de microélectrodes, comprenant
a. un support d'électrodes (1) lequel supporte une pluralité de microélectrodes (2) disposées sur la surface supérieure du support d'électrodes (1),
b. une lamelle couvre-objet (6) avec une surface sur laquelle des cellules électroconductrices (7) sont collées, où les microélectrodes (2) sont configurées pour transmettre un signal électrique aux cellules électroconductrices (7) ou recevoir un signal électrique provenant des cellules électroconductrices (7),
c. une chambre (8) laquelle reçoit le support d'électrodes (1) et la lamelle couvre-objet (6) portant les cellules électroconductrices (7),
**caractérisé en ce que** la lamelle couvre-objet (6) portant les cellules électroconductrices (7) est placée à l'envers sur le support d'électrodes (1) de telle sorte que les cellules électroconductrices (7) sont orientées vers les microélectrodes (2) et que les cellules électroconductrices (7) sont en contact électrique avec la pluralité de microélectrodes (2) afin de recevoir et de transmettre des signaux électriques, où le côté de fond de la lamelle couvre-objet (6) qui comporte les cellules électroconductrices (7) et la surface supérieure du support d'électrodes (1) forment un canal d'écoulement (5) dans la chambre (8) afin de transporter des fluides vers et depuis les cellules électroconductrices (7).

2. Réseau de microélectrodes selon la revendication 1, dans lequel le fond de la chambre (8) forme le support d'électrodes (1).

3. Réseau de microélectrodes selon la revendication 1, dans lequel le canal d'écoulement (5) de la chambre (8) est relié de manière fluidique à un conduit d'alimentation (14) et à un conduit d'évacuation (9).

4. Réseau de microélectrodes selon la revendication 3, dans lequel le conduit d'alimentation (14) et/ou le conduit d'évacuation (9) relient des chambres adjacentes (8) pour former un système multichambre, où chaque chambre (8) est individuellement équipée d'un support d'électrodes (1), d'une lamelle couvre-objet (6) et d'un canal d'écoulement (5) formé par le côté de fond de la lamelle couvre-objet (6) et la surface supérieure du support d'électrodes (1).

5. Réseau de microélectrodes selon l'une quelconque des revendications 1 à 4, dans lequel le côté de fond de la lamelle couvre-objet (6) comprend en outre des éléments d'espacement (13) pour maintenir une distance (A) entre le côté de fond de la lamelle couvre-objet (6) et la surface supérieure du support d'électrodes (1) afin de former le canal d'écoulement (5).

6. Réseau de microélectrodes selon l'une quelconque des revendications 1 à 5, dans lequel la lamelle couvre-objet (6) comprend une ou plusieurs conduites (12) formées au niveau de la surface extérieure de la lamelle couvre-objet (6) afin de transporter les fluides jusque dans le canal d'écoulement (5) formé par la lamelle couvre-objet (6) et le support d'électrodes (1).

7. Réseau de microélectrodes selon l'une quelconque des revendications 1 à 6, dans lequel la chambre (8) comprend un adaptateur d'alimentation (25) constitué d'une pièce rapportée (24) à insérer dans la chambre (8) et d'une partie supérieure (21), laquelle intègre un tube (11) pour transporter le fluide jusque dans ou depuis la chambre (8).

8. Réseau de microélectrodes selon la revendication 7, dans lequel plusieurs chambres (8) sont reliées pour former un système multichambre, et chaque chambre (8) est équipée d'un adaptateur d'alimentation individuel (25), où au moins une chambre (8.1) comprend en outre un bouchon raccord (20) présentant un canal à fluide intégré en son sein (26) lequel est relié de manière fluidique avec le tube (11) de l'adaptateur d'alimentation (25) d'une chambre adjacente (8.2) via un tube de raccordement (15).

9. Réseau de microélectrodes selon la revendication 8, dans lequel le système multichambre comprend en outre une pompe laquelle fait circuler le fluide d'une première chambre (8.1) vers une chambre adjacente (8.2).

10. Réseau de microélectrodes selon l'une quelconque des revendications 7 à 9, dans lequel l'adaptateur d'alimentation (25) est tenu par une bague d'espacement (22) pour maintenir une distance (A) entre le côté de fond de la lamelle couvre-objet (6) et la surface supérieure du support d'électrodes (1) afin de former le canal d'écoulement (5).

11. Réseau de microélectrodes selon la revendication 10, dans lequel plusieurs bagues d'espacement (22) sont montées sur une plaque de recouvrement (23), chaque bague d'espacement (22) est prête pour l'assemblage d'un adaptateur d'alimentation (25) et, si applicable, d'un bouchon raccord (20).

12. Réseau de microélectrodes selon la revendication 7, dans lequel l'adaptateur d'alimentation (25) est tenu par un élément de fixation (30) lequel comprend une partie d'attache (27) laquelle fixe la pièce rapportée (24) de l'adaptateur d'alimentation (25).

13. Réseau de microélectrodes selon la revendication 12, dans lequel une bague d'espacement (22) sert de châssis à l'adaptateur d'alimentation (25), et l'élément de fixation (30) fixe la bague d'espacement (22).

14. Procédé de stimulation extracellulaire ou d'enregistrement de l'activité électrique de cellules électrogènes, comprenant les étapes suivantes consistant à :
a. fournir une pluralité de microélectrodes sur la surface supérieure d'un support d'électrodes,
b. fournir des cellules électroconductrices ou un tissu cellulaire électroconducteur collé(es) sur une lamelle couvre-objet,
c. mettre en contact les cellules électroconductrices avec les microélectrodes, où les microélectrodes transmettent des signaux électriques aux cellules électroconductrices ou reçoivent des signaux électriques provenant des cellules électroconductrices,
d. placer la lamelle couvre-objet portant les cellules électroconductrices à l'envers sur le support d'électrodes de telle sorte que les cellules électroconductrices sont orientées vers les microélectrodes et que les cellules électroconductrices sont en contact électrique avec la pluralité de microélectrodes afin de transmettre ou de recevoir des signaux électriques,
e. former un canal d'écoulement dans une chambre afin de transporter des fluides vers ou depuis les cellules électroconductrices en gardant une distance (A) entre le côté de fond de la lamelle couvre-objet laquelle porte les cellules électroconductrices et la surface supérieure du support d'électrodes.

15. Procédé selon la revendication 14, dans lequel l'activité électrique des cellules électroconductrices est mesurée par un réseau de microélectrodes selon l'une quelconque des revendications 1 à 13.
